## Europäisches Patentamt

## European Patent Office

## Office européen des brevets

(19)

(11) Veröffentlichungsnummer: **0 280 732**
**B1**

(12)

# EUROPÄISCHE PATENTSCHRIFT

(45) Veröffentlichungstag der Patentschrift: **31.10.90**

(51) Int. Cl.⁵: **A 61 L 17/00**

(21) Anmeldenummer: **86905483.3**

(22) Anmeldetag: **04.07.86**

(86) Internationale Anmeldenummer:
**PCT/SU86/00072**

(87) Internationale Veröffentlichungsnummer:
**WO 88/00062 14.01.88 Gazette 88/02**

(54) **NAHTMATERIAL.**

(43) Veröffentlichungstag der Anmeldung:
**07.09.88 Patentblatt 88/36**

(45) Bekanntmachung des Hinweises
auf die Patenterteilung:
**31.10.90 Patentblatt 90/44**

(84) Benannte Vertragsstaaten:
**BE CH DE FR GB IT LI LU NL SE**

(56) Entgegenhaltungen:
**DE-A-2 039 075**
**FR-A-2 013 598**
**FR-A-2 293 947**
**US-A-3 896 813**
**US-A-3 987 797**

(73) Patentinhaber: **VSESOJUZNY NAUCHNO-
ISSLEDOVATELSKY I ISPYTATELNY INSTITUT
MEDITSINSKOI TEKHNIKI
ul. Kasatkina, 3
Moscow, 129301 (SU)**

(72) Erfinder: **BELYKH, Sergei Ivanovich
2-i Krestovsky per., 4-66
Moscow, 129041 (SU)**
Erfinder: **DAVYDOV, Anatoly Borisovich
ul. Krasny Kazanets, 19-1-283
Moscow, 111395 (SU)**
Erfinder: **MOSCHENSKY, Anatoly Denisovich
Bykovskoe shosse, 41-66
Moskovskaya obl., Malakhovka, 140009 (SU)**
Erfinder: **KANSHIN, Nikolai Nikolaevich
ul. Malaya Filevskaya, 68-10
Moscow, 121433 (SU)**
Erfinder: **KOVALENKO, Igor Leonidovich
ul. Chapaeva, 7-30
Khimki Moscow, 141400 (SU)**
Erfinder: **KIRILLOV, Jury Borisovich
ul. Dzerzhinskogo, 73-6
Ryazan, 390005 (SU)**
Erfinder: **OSIPOV, Gennady Ionovich
ul. Studencheskaya, 34-9
Moscow, 121165 (SU)**

Courier Press, Leamington Spa, England.

**EP  0 280 732  B1**

Erfinder: **UTYAMYSHEV, Rustam Ismailovich
pr. Mira, 118-222
Moscow, 129164 (SU)**

Vertreter: **von Füner, Alexander, Dr. et al
Patentanwälte v. Füner, Ebbinghaus, Finck
Mariahilfplatz 2 & 3
D-8000 München 90 (DE)**

# EP 0 280 732 B1

**Beschreibung**

Die vorliegende Erfindung bezieht sich auf die Medizintechnik und betrifft insbesondere ein Nahtmaterial.

Bekannt sind nicht resorbierbare gegen Mikroben wirksame Nahtmaterialien, beispielsweise auf der Basis von Polyethylenterephthalat oder Polypropylen, mit dem daran chemisch gebundenen Antimikrobenpräparat Geomyzin. Diese Nahtmaterialien bewirken eine feste Fixation von Weichteilen und wirken gegen Mirkoben innerhalb von 2 Tagen (A. A. Shalimov u.a. Polimery v meditsine. 1977, Bd. VII, Nr. 1, S. 19—26). Fäden aus solchem Nahtmaterial können jedoch dem Abbau auf biologischem Wege nicht unterworfen werden, und die Zeit ihrer antimikrobiellen Wirkung ist sehr begrenzt.

Bekannt sind Nahtmaterialien auf der Basis von bioresorbierbarem Polymer, beispielsweise Oxycellulosefasern mit Sulfonylamidpräparaten, welche an die Grundlage über kovalente Bindungen gebunden sind. Als Sulfonamidpräparat enthält dieses Nahtmaterial beispielsweise 2-(para-Aminobenzosulfamido)-5-ethyl-1,3,4-thiadiazol oder O-3-Amino-3-desoxy-α-D-glukopyranosil-(1→6)-O-[6-amino-6-desoxy-α-D-glukopyranosil(1→]-2-desoxy-D-streptomyzin (Kanamysin) (SU,A 338525). Die Fäden aus dem angegebenen Nahtmaterial können unter Einwirkung von Medien im Organismus einem biologischen Abbau unterzogen werden und wirken gegen Mikroben innerhalb von 16 Tagen. Diese Fäden besitzen jedoch keine ausreichende Festigkeit, und die Anzahl von antibakteriellen Mitteln, die an die Hauptkette des Nahtmaterials chemisch gebunden werden können, ist sehr beschränkt. Im Laufe der chemischen Anlagerung wird das Molekül des antimikrobiellen Präparats durch chemische Reagenzien angegriffen, was die teilweise Zerstörung des Moleküls hervorrufen kann, wodurch die antimikrobielle Wirkung herabgesetzt wird oder völlig verschwindet.

Der Erfindung liegt eine Aufgabe zugrunde, durch Modifikation der Grundlage ein Nahtmaterial herzustellen, welches eine hohe Festigkeit besitzt, beliebige Arten von Antimikrobenpräparaten enthält und einen breiten Zeitraum der antimikrobiellen Wirkung sichert.

Die Aufgabe wird dadurch gelöst, daß das erfindungsgemäße Nahtmaterial, welches die Grundlage aus einem bioresorbierbaren Polymer und ein Antimikrobenpräparat beinhaltet, erfindungsgemäß eine auf dei Grundlage aufgebrachte Schicht aus einem copolymer von N-Vinylpyrrolidon mit Alkylacrylat und/oder Alkylmethacrylat enthält, welche ein Präparat mit einem oder mehreren antimikrobiell wirkenden Stoffen in einer Menge von 1 bis 40 Gew.-% berechnet auf die aufgebrachte Schicht, enthalt, wobei dei Dicke dieser Schicht zwischen 10 und 100% der Dicke der Grundlage liegt.

Das erfindungsgemäße Nahtmaterial besitzt eine Reihe von Vorteilen im Vergleich zum bekannten Nahtmaterial und zwar

— die Bruchfestigkeit des erfindungsgemäßen Nahtmaterials übersteigt die des bekannten Nahtmaterials um 15 bis 20%

— die Festigkeit der Knopfnahtverbindung liegt über 25 bis 30% höher als die des bekannten Nahtmaterials

— das erfindungsgemäße Nahtmaterial ermoglicht die Verwendung von beliebigen Arten der Antimikrobenpräparate darunter auch solche gegenüber allen Wirkungstypen empfindlichen Präparate wie Chinoxalinderivate

— die Zeit der antimikrobiellen Einwirkung in der Schädigungszone kann in einem weiten Bereich (von 3 bis 20 Tagen) geregelt werden.

Das erfindungsgemäß Nahtmaterial enthält eine auf die Grundlage aufgebrachte Schicht aus einem Copolymer von N-Vinylpyrrolidon mit Alkylacrylat und/oder Alkylmethacrylat, welche ein Antimikrobenpräparat oder ein Gemisch von Präparaten enthält.

Als Antimikrobenpräparate können beliebige Präparate, zum Beispiel Antibiotika, Sulfonylamidpräparate, Derivate von Chinoxalin, Nitrofuran, Hydroxychinolin u.a. enthalten sein.

Die auf die Grundlage aufgebrachte Schicht weist eine kürzere Dauer des abbaus gegenüber der Grundlage auf. Die Dicke dieser Schicht liegt zwischen 10 und 100% der Dicke der Grundlage.

Bei einer Schichtdicke, die unter 10% der Dicke der Grundlage liegt, wird die Technologie der Beschichtung erschwert, und die mechanischen Kennwerte werden verschlechtert, während die Menge des Antimikrobenpräparats so gering wird, daß die antimikrobielle Wirkung nicht gewährleistet wird. Bei einer Schichtdicke, die über 100% der Dicke der Grundlage liegt, werden die Festigkeitseigenschaften des Nahtmaterials ebenfalls verschlechtert, was dazu führt, daß sich die aufgebrachte Schicht während der Aufbewahrung schichtenweise ablöst und von der Grundlage abgetrennt wird, die Fadensteifigkeit bedeutend zunimmt und die Oberflächenglätte beeinträchtigt wird.

Bei der Verwendung des Nahtmaterials kommt es durch die chirurgische Naht unter Einwirkung von flüssigen Medien des Organismus zur Diffusion des Antimikrobenpräparats durch den Film des auf die Grundlage aufgebrachten Copolymers; die Zeit seiner Absonderung in die umgebenden Gewebe hängt dabei von der Filmdicke des Copolymers ab. Die Gesamtmenge des Antimikrobenpräparats kann im voraus im Stadium der Zubereitung der Mischung zum Aufbringen auf die Grundlage in einem weiten Bereich variert werden, und die aufzubringende Schicht, die das Antimikrobenpräparat enthält, setzt die physikalisch-mechanischen Eigenschaften des Nahtmaterial nicht herab. Dadurch, daß das Copolymer, enthaltend ein Antimikrobenpräparat, einen kürzeren Bioabbau gegenüber der Grundlage aufweist, bleibt die Nahtfestigkeit auf dem erforderlichen Niveau innerhalb der ganzen Absonderungszeit des

3

EP 0 280 732 B1

Antimikrobenpräparats erhalten.

Es wurden die Prüfungen des erfindungsgemäßen Nahtmaterials in vivo und in vitro durchgeführt.

Durch Untersuchung der Eigenschaften von Fäden, überzogen mit dem Film aus Copolymer von N-Vinylpyrrolidon mit Alkylmethacrylat, ist festgestellt worden, daß das Aufbringen des Polymers mit dem Antibiotikum auf den säurebehandelten Kapronfaden die Erhöhung der Bruchfestigkeit des Fadens Nr. 1 (93,5 tex) von 5,7 auf 6,1 kg bewirkt, während die Festigkeit der Knopfnahtverbindung von 3,6 auf 4,4 kg ansteigt.

Die gleichen Ergebnisse wurden auch bei nassen Fäden gewonnen. Die Bruchfestigkeit des Fadens vor und nach Aufbringen des polymeren Überzugs beträgt in diesem Fall 4,3 bzw. 5,1 kg und die Festigkeit der Kopfnahtverbindung 2,2 bzw. 3,2 kg.

Folgende Ausführungsformen des Nahtmaterials waren klinisch untersucht: 1) Nahtmaterial auf der Basis von säurebehandeltem Kapron mit einem Überzug aus dem Copolymer von N-Vinylpyrrolidon mit Butylmethacrylat, welches 22 Gew.% 1,4-Di-N-oxid-2,3-bis(hydroxymethyl)chinoxalin enthält, wobei die Dicke der aufgebrachten Schicht 24% der Dicke der Grundlage beträgt, was einem Fadengehalt an Antimikrobenpräparat von 6 Gew.% entspricht; 2) Nahtmaterial auf der Basis von säurebehandeltem Kapron, beschichtet mit dem ähnlichen Copolymer, welches 10 Gew.% 1,4-Di-N-oxid-2,3-bis(hydroxymethyl)chinoxalin und 21 Gew.% 1,4-Di-N-oxid-2,3-bis(acetoxymethyl)chinoxalin enthält, wobei die Dicke der aufgebrachten Schicht 23°% der Dicke der Grundlage beträgt, was einem Fadengehalt am ersten Antimikrobenpräparat von 1,9 Gew.% und am zweiten Antimikrobengehalt von 3,9 Gew.% entspricht; 3) Nahtmaterial auf der Basis von säurebehaldeltem Kapron, beschichtet mit dem ähnlichen Copolymer, welches 32 Gew.% Gentamyzinsulfat enthält, wobei die Dicke der aufgebrachten Schicht 14% der Dikke der Grundlage beträgt, was einem Fadengehalt am Antimikrobenpräparat von 4,5 Gew.% entspricht. Das angegebene Nahtmaterial wurde zum Anlegen von Anastomosen im Magendarmtrakt bei 29 Patienten, im Falle der akuten Appendizitis bei 17 Patienten, der kalkulösen Cholezystitis bei 19 Patienten, des Magengeschwürs bei 26 Patienten, des Magenkrebs bei 11 Patienten und der Varikosität von Extremitäten bei 22 Patienten verwendet. In allen Fällen sind die sichere Fixation von Geweben und keine eiterhafte Komplikationen bei postoperativen Wunden und Darmanastomosen zu vermerken.

Das erfindungsgemäße Nahtmaterial zusammen mit Verbindungselementen für innere Organe wurde in der kinderchirurgischen Praxis bei Operationen an parenchymatösen Organen in 27 Fällen (Einnähen von Leber- und Darmgeweben) und bei Organe erhaltenden Operationen (Milz) an 17 Kranken geprüft. Komplikationen, verbunden mit der postoperativen Undichtigkeit von Nähten, oder eiterhafte Komplikationen traten nicht auf. Der Untersuchungsbefund von Blut und Harn zeigt Normalwerte.

Das erfindungsgemäße Nahtmaterial auf der Basis von säurebehandeltem Kapron, beschichtet mit dem Copolymer aus N-Vinylpyrrolidon mit Butylmethacrylat, welches 6 Gew.% 1,4-Di-N-oxid-2,3-bis-(hydroxymethyl)-chinoxalin enthält, wurde zum Nähen der längslaufenden Sägeschnittstelle des Brustbeins nach der Herzoperation untersucht. Anstatt des gewöhnlich verwendbaren Stahldrants wurden 5 bis 6 Z-förmige Nähte auf das Brustbein angelegt. Die Wundheilung und die Brustbeinverwachsung dauerte wie üblich. Komplikationen anläßlich der Vereiterung der Nähte wurden nicht nachgewiesen. Festgestellt ist keine Schmerzempfindung bei Bewegungen, was im Falle der Verwendung von Drahtschnürungen gewöhnlich zum Ausdruck kommt. Ausgeschlossen ist die Durchführung der wiederholten Operation anläßlich der Entfernung von Drahtschnürungen.

Das erfindungsgemäße Nahtmaterial wurde ebenfalls bei der Plastik im Bereich des harten und weichen Gaumens geprüft. Die Besonderheiten dieser Operation bestehen darin, daß hier wegen Spezifität eine längere Heilungsdauer (12 bis 18 Tage) in Rechnung gezogen wird und daß die Heilung unter ständiger Infektion und Einwirkung von flüssigen Medien in der Mundhöhle stattfindet. Die Beobachtung von 24 Kranken hat· ergeben, daß es in allen Fällen zur primären Wundheilung ohne jegliche Komplikationen kommt.

Bei 35 Kranken wurde die Osteosynthese des Kiefers unter Verwendung des erfindungsgemäßen Nahtmaterials dann verwirklicht, wenn eine wesentliche Verschiebung der Frakturenden nicht auftrat. Die Festigkeitseigenschaften des Nahtmaterials waren vollkommen ausreichend, und das Vorliegen des antimikrobiellen Überzugs ermöglichte, in 11 Fällen diese Nahttechnik bei offenem Bruch mit Erfolgt zu verwenden.

Zum besseren Verstehen der vorliegenden Erfindung werden folgende Beispiele für das erfindungsgemäße Nahtmaterial angeführt.

## Beispiel 1

Nahtmaterial, bestehend aus 93,5 tex feinem Kapronfaden als Grundlage und auf die Grundlage aufgebrachter Schicht von Copolymer aus Butylmethacrylat mit N-Vinylpyrrolidon und enthaltend 6% 1,4-Di-N-oxid-2,3-bis(hydroxymethyl)-chinoxalin, bezogen auf das Fadengewicht. Die Dicke der aufgebrachten Schicht beträgt 24% der Dicke der Grundlage. Man stellt das angegebene Material dadurch her, daß man auf den genannten Kapronfaden die Lösung von Copolymer aus Butylmethacrylat mit N-Vinylpyrrolidon in Ethylalkohol (15 g Copolymer in 100 ml Ethylalkohol) aufbringt, welche 4,2 g 1,4-Di-N-oxid-2,3-bis-(hydroxymethyl)chinoxalin (22%, bezogen auf das Copolymergewicht) enthält. Der Faden wird getrocknet.

Es wurden die Untersuchungen am angegebenen Nahtmaterial durchgeführt. Die Prüfergebnisse gibt nachstehend angeführte Tabelle an.

4

Beispiel 2

Nahtmaterial, bestehend aus 48 tex feinem Faden aus Polyvinylalkohol als Grundlage und auf die Grundlage aufgebrachter Schicht von Copolymer aus Hexylacrylat mit N-Vinylpyrrolidon und enthaltend 2% Kanamyzin, bezogen auf das Fadengewicht (19%, bezogen auf das Copolymergewicht). Die Dikke der aufgebrachten Schicht beträgt 10% der Dicke der Grundlage. Man stellt das angegebene Nahtmaterial analog zu dem in Beispiel 1 beschriebenen bei einem Gewichtsverhältnis von Copolymer, Präparat und Lösungsmittel wie 10:2,1 bzw. 80 her. Die Prüfergebnisse des angegebenen Nahtmaterials sind in der nachstehend angeführten Tabelle dargestellt.

Beispiel 3

Nahtmaterial, bestehend aus 50,6 tex feinem Faden aus Monocarboxycellulose als Grundlage und auf die Grundlage aufgebrachter Schicht von copolymer aus N-Vinylpyrrolidon mit Methylmethacrylat und aus N-Vinylpyrrolidons mit Ethylacrylat im Verhältnis von 1:1 und enthaltend 0,1% Gentamyzinsulfat, bezogen auf das Fadengewicht, und 0,40% 4-(para-Aminobenzosulfamido)-6-methoxypyromidin (1%, bezogen auf das Copolymergewicht). Die Dicke der aufgebrachten Schicht ist dem Durchmesser der Grundlage gleich. Man stellt das Nahtmaterial analog zu dem in Beispiel 1 beschriebenen bei einem Gewichtsverhältnis von Copolymer, Gemisch von Praparaten und Lösungsmittel wie 100:0,5 bzw. 95 her. Die Prüfergebnisse des angegebenen Materials gibt die nachtstehend angeführte Tabelle an.

Beispiel 4

Nahtmaterial, bestehend aus 93,5 tex feinem Kapronfaden als Grundlage und auf die Grundlage aufgebrachter Schicht aus Copolymer aus N-Vinylpyrrolidon mit Butylmethacrylat und enthaltend 3,12% 1,4-Di-N-oxid-2,3-bis (hydroxymethyl)chinoxalin, bezogen auf das Fadengewicht, und 4,22% 1,4-N-oxid-2,3-bis-(acetoxymethyl)chinoxalin, bezogen auf das Fadengewicht (40%, bezogen auf das Copolymeragewicht). Die Dicke der aufgebrachten Schicht beträgt 12% der Dicke der Grundlage. Man stellt das Nahtmaterial analog zu dem in Beispiel 1 beschriebenen bei einem Gewichtsverhältnis von Copolymer, Gemisch von Präparaten und Lösungsmittel wie 10,5:7 bzw. 112 her. Die Prüfergebnisse sind in der nachstehend angeführten Tabelle dargestellt.

Beispiel 5

Nahtmaterial, bestehend aus 93,5 tex feinem Kapronfaden als Grundlage und auf die Grundlage aufgebrachter Schicht von Copolymer analog zu Beispiel 1 und enthaltend 4,5% Gentamyzinsulfat, bezogen auf das Fadengewicht (24%, bezogen auf das Copolymergewicht). Die Dicke der aufgebrachten Schicht beträgt 11% der Dicke der Grundlage.

Man stellt das Nahtmaterial analog zu dem in Beispiel 1 beschriebenen bei einem Gewichtsverhaltnis von Copolymer, Präparat und Lösungsmittel wie 13,5:4,5 bzw. 100 her. Die Prüfergebnisse des angegebenen Nahtmaterials sind in der nachtsthend angeführten Tabelle dargestellt.

TABELLE

Vergleichseigenschaften des erfindungsgemäßen Nahtmaterials und der bekannten Nahtmaterialien

| Lfd. Nr. | Fadenart | Ausgangskennwerte spezifische Festigkeit in p/tex | spezifische Festigkeit der Knofnahtverbindung in p/tex | Kennwerte nach 10 Tagen Verweillen in weichen Geweben | | | |
|---|---|---|---|---|---|---|---|
| | | | | spezifische Festigkeit in p/tex | spezifische Festigkeit der Knopfnahtverbindung in p/tex | Maximale Nachweiszeit für Präparat im Blut in Tagen | Verhältnis zwischen Biodestruktionszeiten der aufgebrachten Schicht und der Grundlage |
| 1 | 2 | 3 | 4 | 5 | 6 | 7 | 8 |
| 1 | das erfindungsgemäße Nahtmaterial nach Beispiel 1 | 69 | 58 | 62 | 45 | 10 | 0,3—0,4 |
| 2 | das erfindungsgemäße Nahtmaterial nach Beispiel 2 | 78 | 75 | 52 / 11 | 42 / 7 | 7 / 12—14 | 0,7—0,8 / 0,7—0,8 |
| 3 | das erfindungsgemäße Nahtmaterial nach Beispiel 3 | 32 | 25 | 63 | 46 | 18—20 | 0,3—0,4 |
| 4 | das erfindungsgemäße Nahtmaterial material nach Beispiel 4 | 73 | 59,5 | 62 / 6 | 45 / 2 | 10 / 10 | 0,3—0,4 / — |
| 5 | das erfindungsgemäße Nahtmaterial nach Beispiel 5 | 70 | 60 | 60 | 38 | — | — |
| 6 | das bekannte Nahtmaterial auf der Basis von Oxycellulosefasern, enthaltend Kanamyzin | 18 | 15 | | | | |
| 7 | das bekannte Nahtmaterial Kapronfaden | 63 | 40 | | | | |

EP 0 280 732 B1

# EP 0 280 732 B1

Das erfindungsgemäße Nahtmaterial dient zur Fixation von Weichteilen bei beliebigen chirurgischen Eingriffen und vor allem unter Bedingungen der möglichen Infektion der umgebenden Gewebe.

**Patentanspruch**

Nahtmaterial, welches die Grundlage aus einem bioresorbierbaren Polymer und ein Antimikrobenpräparat beinhaltet, dadurch gekennzeichnet, daß es ein auf die Grundlage aufgebrachte Schicht aus einem Copolymer von N-Vinylpyrrolidon mit Alkylacrylat und/oder Alkylmethacrylat enthält, welche ein Präparat mit einem oder mehreren antimikrobiell wirkenden Stoffen in einer Menge von 1 bis 40 Gew.-%, berechnet auf die aufgebrachte Schicht, enthält, wobei die Dicke dieser Schicht zwischen 10 und 100% der Dicke der Grundlage liegt.

**Revendication**

Matériau pour ligatures qui contient une base d'un polymère biorésorbable et d'un préparation antimicrobienne, caractérisé en ce qu'il contient une couche placée sur la base, en un copolymère de N-vinylpyrrolidone avec de l'acrylate d'alkyle et/ou du méthacrylate d'alkyle qui contient une préparation avec une ou plusieurs matières antimicrobiennes en une quantité de 1 à 40% en poids en se rapportant à la couche, l'épaisseur de ladite couche étant comprise entre 10 et 100% de l'épaisseur de la base.

**Claim**

Suture material comprising a base made of a bioresorbable polymer and an antimicrobial preparation, characterised in that the said suture material comprises a layer applied to the base made of a copolymer of N-vinylpyrrolidone with alkylacrylate and/or alkylmethacrylate, which contains a preparation with one or more microbially acting substances in a quantity of 1 to 40% by weight relative to the applied layer, the thickness of the said layer being between 10 and 100% of the thickness of the base.